(19) Europäisches Patentamt
European Patent Office
Office européen des brevets

(11) Publication number : 0 299 995 B1

# (12) EUROPEAN PATENT SPECIFICATION

(45) Date of publication of patent specification : 30.10.91 Bulletin 91/44

(51) Int. Cl.[5] : C01B 3/06, C01B 3/02

(21) Application number : 88900855.3

(22) Date of filing : 11.01.88

(86) International application number : PCT/NO88/00003

(87) International publication number : WO 88/05422 28.07.88 Gazette 88/17

## (54) PROCESS FOR CONVERTING CARBON MONOXIDE AND WATER TO HYDROGEN AND CARBON DIOXIDE.

(30) Priority : 27.01.87 NO 870330
12.08.87 NO 873375

(43) Date of publication of application : 25.01.89 Bulletin 89/04

(45) Publication of the grant of the patent : 30.10.91 Bulletin 91/44

(84) Designated Contracting States : AT BE CH DE FR GB IT LI LU NL SE

(56) References cited :
US-A- 3 262 973
US-A- 4 067 958
US-A- 4 137 298
US-A- 4 145 405

(73) Proprietor : ONSAGER, Olav-T.
Institutt fo Industriell Kjemi Sem Saelands vei 4
N-7034 Trondheim (NO)

(72) Inventor : ONSAGER, Olav-T.
Institutt fo Industriell Kjemi Sem Saelands vei 4
N-7034 Trondheim (NO)

(74) Representative : Holmes, Michael John et al
Frank B. Dehn & Co. European Patent Attorneys Imperial House 15-19 Kingsway
London, WC2B 6UZ, (GB)



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

It has been known for a long time that salts of formic acid, socalled formates, may be used as reducing agents in organic as well as inorganic chemical reactions. It has recently been demonstrated that sodium and potassium formate dissolved in water, in the presence of a heterogeneous noble metal catalyst such as palladium on active carbon, is decomposed to hydrogen and bicarbonate in a stoichiometric reaction in which primarily one mole of hydrogen and one mole of bicarbonate are formed per mole of reacted sodium or potassium formate (H. Wiener et al, J. Mol. Cat. 35 (1986) 277-284)).

The opposite reaction, viz. that sodium and potassium formate may be prepared by palladium catalyzed hydrogenation of the corresponding bicarbonate compounds, has been known for a long time (D. P. 283.895 (1913) and G. Breidig and S. R. Carter Ber. 47 (1914), 545)). Thus, in practice reaction (1) has been demonstrated to be an equilibrium reaction:

$$HCOO^- + H_2O \rightleftharpoons HCO_3^- + H_2 \qquad (1)$$

The presence of $Na^+$ or $K^+$ in water has the effect that the solution is slightly basic.

When considered separately, the industrial importance of reaction 1 is very limited. Within the scope of the present invention there is provided an advantageous process in which reaction 1 represents a part, and in which the by-product, bicarbonate, in reaction with carbon monoxide is reconverted to formate for repeated use in the formation of hydrogen.

It is well known that bicarbonate upon thermal decomposition leads to the formation of carbonate, water and carbon dioxide as illustrated in reaction 2

$$2\,HCO_3^- \rightarrow CO_3^{--} + H_2O + CO_2 \qquad (2)$$

and that carbonate in reaction with water and carbon monoxide may result in the formation of bicarbonate and formate as products in accordance with reaction 3.

$$CO_3^{--} + H_2O + CO \rightarrow HCO_3^- + HCOO^- \qquad (3)$$

In combination, reactions 1 + 2 + 3 will give the total conversion which is illustrated in reaction 4, a reaction which is of great industrial importance.

$$CO + H_2O \rightarrow H_2 + CO_2 \qquad (4)$$

Further, it is known that reactions 1, 2 and 3 may be carried out in water as solvent and that these may be combined to a cyclic process as described in US patent No. 4.137.298. As alkali metal sodium or potassium is then used. A clear disadvantage of water as solvent is that the formation rate for formate in accordance with reaction 3 is very low, so that use of relatively high temperatures and CO pressures above 20 bar is necessary, which makes the process uneconomic and energy consuming.

In spite of the fact that the reactions 2 and 3 together lead to the formation of formate, from inexpensive and readily available starting materials, the industrial production of lithium, sodium and potassium formate takes place primarily by reacting the corresponding hydroxide compounds with carbon monoxide. US patent 3.262.973 illustrates a process in which lithium, sodium or potassium hydroxide dissolved in an organic solvent such as ethanol, methanol, isopropanol or butanol, is reacted with carbon monoxide to give lithium, sodium and potassium formate respectively. Here the highest yields of formate salt are obtained in the sodium system (i.e. with sodium hydroxide), with ethanol as solvent.

On the basis of analogy considerations it would therefore seem obvious for a skilled person to try and use sodium carbonate dissolved in a mixture of alcohol and water in reaction with carbon monoxide, in order to prepare sodium formate as product. However, the results from such experiments illustrate that the rate of formation of sodium formate from sodium carbonate both by using ethanol, methanol, isopropanol and butanol, is very low, which should indicate that carbonate and carbon monoxide are not suitable in the preparation of formate.

In contrast to what might be expected, it has now surprisingly been found that potassium, rubidium and cesium carbonate in methanol may be reacted with carbon monoxide with the formation of potassium, rubidium and cesium formate respectively at a rate which under equal conditions, is more than an order of magnitude higher than that which is obtained with the homologous sodium system, and that such results are only obtained when methanol is used as a major component of the liquid reaction medium ie. when the reaction medium comprises at least 50% by weight of methanol. Further, it has surprisingly been found that high formation rates of potassium formate in an unusual manner is dependant on the fact that the molar ratio between water and potassium carbonate present in the reaction mixture, is kept within certain limits, high formation rates requiring a molar ratio between water and carbonate of less than 1.5, but above 0.2, said ratio preferably being about 1, or said ratio being above 4 but less than 20.

At approximately stoichiometric ratios between water and potassium carbonate and a reaction temperature of 100°C, the reaction rate is so high that it is probably limited by the diffusion rate of carbon monoxide in the system at a pressure of 10 bar CO.

In contrast therewith, the reaction of potassium, rubidium and cesium carbonate with CO in such systems using ethanol, isopropanol or butanol as solvent, takes place at a very low rate.

In addition to methanol the liquid solvent may of course contain minor amounts of organic solvent, such as tertiary organic amines or linear and cyclicethers, without any essential reduction of the reaction rate.

When rubidium or cesium carbonate is used, it has been found that the highest formation rates for formate in all the systems are obtained when the molar ratio between water and carbonate is less than 20 but above 0.2. On the basis of the new knowledge which has been acquired herein, one has succeeded in the combination of the reactions 1, 2 and 3 in such a manner that a technically/economically advantageous process is obtained for converting carbon monoxide and water to hydrogen and carbon dioxide. A particularly advantageous feature of the process according to the invention is that all three reactions 1, 2 and 3, are carried out in essentially the same type of liquid reaction medium, viz. a mixture of methanol and water in which methanol represents the major component. A consequence of this is that in those cases where the reactions 1-3 take place in separate vessels, the total process may be carried out in a particularly energy saving manner.

The process according to the invention is characterized by the features given in claim 1.

In practice, the process according to the invention can be carried out in several manners. It may be carried out in a process in which the reactions 1, 2 and 3 entirely or partly take place in separate reaction zones, and it may be carried out continuously or discontinuously. Potassium, rubidium or cesium compounds in the form of formate, bicarbonate and carbonate appear as products and reactants at the different process stages, and methanol is used as solvent.

The formation of hydrogen according to reaction 1 is performed in methanol at a temperature in the range 50-240°C and in the presence of a heterogeneous palladium catalyst. Surprisingly high reaction rates are obtained herein with methanol as solvent. The concentrations of catalyst, formate and water are chosen so that a suitable formation rate of hydrogen is obtained. At a temperature below about 150°C, hydrogen is primarily formed as a gaseous product. A particularly pure hydrogen product is formed if the temperature is lower than about 150°C and the reaction mixture in addition to formate contains a carbonate compound such as potassium, rubidium or cesium carbonate. At a temperature above about 150°C, carbon dioxide will also be formed as product according to reaction 2 in addition to hydrogen. Thus, the reaction of bicarbonate to carbonate, water and carbon dioxide may, if desired, be carried out simultaneously with reaction 1. If an embodiment in which reaction 2 is to take place in a separate reaction zone is selected, a temperature above about 150°C is chosen in this zone.

An essential feature of the process according to the invention is that the palladium catalyst essentially remains in the reaction zone in which hydrogen is formed.

The formation of formate, according to reaction 3, is performed in methanol at a temperature in the range 50°C to 240°C with water present within the limits defined above. If a temperature lower than about 150°C is selected, reaction 3 will essentially be the only one which takes place, while a temperature above about 150°C will also in this case have the effect that reaction 2 takes place simultaneously with the formation of formate. The upper temperature limit of 240°C is such that an essential part of the methanol present in the reactor, is present in liquid form.

The reactor pressure is less critical than the temperature and is suitably selected so that an essential part of the methanol remains in liquid form, typically within the range 1 bar to 100 bar. The partial pressures of the different gases, carbon monoxide, carbon dioxide and hydrogen, are selected so that suitable reaction rates are obtained. Typically a partial pressure of carbon monoxide in the formation of formate is selected within the range from 1 bar to 50 bar and is preferably a pressure lower than 20 bar.

In the process according to the invention the starting material used may be pure carbon monoxide or carbon monoxide containing gas mixtures, such as carbon monoxide mixed with nitrogen, methane, hydrogen, ethane, ethene and carbon dioxide Practically pure hydrogen, hydrogen mixed with carbon monoxide or hydrogen mixed with carbon dioxide may be recovered as product, or the hydrogen prepared may be used directly, for instance for reduction purposes.

If it should be desirable, reactions 1-3 may be carried out in the same vessel. The temperature in such an embodiment is selected in the range 150-240°C. As product there is recovered a mixture of hydrogen and carbon dioxide, often together with minor amounts of unreacted carbon monoxide.

Pure hydrogen is used on a large scale by the industry, for instance in the production of ammonia or in other known hydrogenation processes. A mixture of hydrogen and carbon monoxide is used in several industrial processes, for instance in the production of methanol, higher alcohols and hydrocarbons.

The following examples illustrate the invention further. All pressures recited in the Examples are absolute

values measured on a gauge.

Example 1

70 ml of methanol, 1.2 g of water, 2.36 g of potassium formate and 0.2 g of Pd(5%)/C (Strem Chemicals Inc., Danvers, Mass. 01923, USA) were charged to a 100 ml steel (316 SS) autoclave equipped with magnetic stirrer, electric heating means and temperature control means, pressure gauge for the range 0.2-40 bar and means for removing gas samles from the gaseous phase in the reactor for gas chromatographic analysis.

The apparatus was filled with inert gas ($N_2$) at room temperature to a pressure of about 10 bar, and the experiment was started by heating the reaction mixture to 140 ± 2°C.

The rate of hydrogen formation was found to be about 5000 moles of $H_2$/mole Pd per 24 hours.

Example 2

70 ml of methanol, 6.0 g of water, 8.0 g of cesium formate and 0.3 g of Pd(5%) on activated carbon (Strem) were charged to a 100 ml steel (316 SS) autoclave equipped with magnetic stirrer, electric heating means and temperature control means, pressure gauge for the range 0.2-40 bar and means for removing gas samples from the gaseous phase in the reactor for gas chromatographic analysis. The apparatus was filled with inert gas ($N_2$) at room temperature to a pressure of about 10 bar, and the experiment was started by heating the reaction mixture up to 170 ± 2°C.

The composition of the gas phase in the reactor was then analyzed with respect to hydrogen as a function of the reaction time. On the basis of the data obtained the initial formation rate of hydrogen was found to be about 7000 moles of $H_2$/mole of Pd per 24 hours. In addition to hydrogen, minor amounts of $CO_2$ are formed as a gaseous product.

Example 3

55.7 ml of methanol, 1.2 g of triethylamine, 9.0 g of $K_2CO_3$ and 1.5 g of water were charged to a 100 ml steel (316 SS) autoclave equipped with magnetic stirrer, electric heating means and temperature control means, manometer (0.2-40 bar) and means for adding CO gas to the gas phase above the liquid reaction mixture. The reaction mixture was heated to 100 ± 2°C and reacted with CO at a CO partial pressure of 10 bar. The CO uptake and the formation rate of potassium formate were found to be about 100 mmoles per hour.

In addition to potassium formate there is also formed potassium bicarbonate as product, and at the same time water is consumed in the reaction. In order to increase the yield of formate, bicarbonate may according to known methods be converted to carbonate (+$CO_2$) which is then reacted with CO to formate.

Example 4

0.7 $dm^3$ of methanol and 138 g of $K_2CO_3$ were charged to a 1 $dm^3$ steel (316 SS) autoclave equipped with feeding tubes for gas and liquid, electric heating means, temperature control means, mechanical stirrer, manometer (0.2-40 bar) and gas outlet via a reflux condenser which was cooled with water to 12°C. The reaction mixture was heated to 160°C under a nitrogen pressure of 30 bar. The reaction was started by continuously injecting a generator gas consisting of 50% CO and 50% $N_2$ into the reaction mixture in an amount of 450 $Ndm^3$ per hour, while at the same time water was pumped into the reactor at a rate of 24 g of $H_2O$ per hour. The reaction temperature during the experiment was 155 ± 5°C. The exhaust gas was analyzed with respect to the content of formed $CO_2$ and unreacted CO by means of known gas chromatographic methods. During a reaction time of 1 hour, 0.75 moles of $CO_2$ were formed, and the degree of conversion of $K_2CO_3$ to potassium formate was found to be 80%. In addition to potassium formate the reaction mixture also contained some unreacted $K_2CO_3$ and minor amounts of $KHCO_3$. The degree of conversion of CO during the experiment was 16%.

This experiment was repeated with the difference that 200 g of $KHCO_3$ was used instead of $K_2CO_3$, so that $K_2CO_3$ and water (+$CO_2$) are primarily formed in the reaction mixture and are further converted with CO to formate. The reaction time was chosen so that about 1.7 moles of $CO_2$ product were formed. The composition of the other reaction products was found to be essentially the same as in the above experiment in which $K_2CO_3$ was used as starting material.

Samples of potassium formate prepared according to the process of examples 3 and 4 were tested in the process for preparing hydrogen according to example 1 and were found to have the same properties as the commercial product.

Example 5

Weighed amounts of sodium, potassium, rubidium or cesium carbonate, methanol, ethanol, isopropanol or n-butanol and water were charged to a 100 ml steel (316 SS) autoclave equipped with magnetic stirrer, electric heating means and temperature control means, manometer (0.2-40 bar) and means for adding nitrogen and CO gas to the gas phase above the liquid reaction mixture, which was first heated to 100°C for 1 hour in the presence of nitrogen gas only (about 3 bar at room temperature). Then the reaction between the carbonate, CO and water with formation of formate was started, by establishing a CO partial pressure of 10 bar in the reactor. The uptake of CO and the initial formation rate of formate ($r_o$, $mM.min^{-1}l^{-1}$) were determined.

The composition of the reaction mixture at the start of the reaction and the rate of formation of formate in the different experiments are given in Table 1.

Table 1 - Test conditions and results

| Test no. | Reaction mixture at start | | | Formation of formate |
|---|---|---|---|---|
| | Carbonate, g | Solvent, g | Water, g | $r_o$, $mM.min^{-1}l^{-1}$ |
| 1 | $Na_2CO_3$, 6.75 | - | 60 | <1 |
| 2 | $K_2CO_3$, 8.80 | - | 60 | <1 |
| 3 | $Na_2CO_3$, 6.75 | MeOH, 45.5 | 1.15 | 2 |
| 4 | " 6.75 | " 45.5 | 4.55 | 1 |
| 5 | $K_2CO_3$, 8.80 | " 45.5 | 1.15 | 30 |
| 6 | " 8.80 | EtOH, 46.0 | 1.15 | 2 |
| 7 | " 8.80 | i-PrOH, 46.0 | 1.15 | 1 |
| 8 | " 8.80 | n-BuOH, 46.0 | 1.15 | 1 |
| 9 | $Rb_2CO_3$, 14.7 | MeOH, 45.5 | 1.15 | 30 |
| 10 | $Cs_2CO_3$, 8.00 | " 45.5 | 0.90 | 29 |
| 11 | " 8.00 | " 45.5 | 0.45 | 30 |
| 12 | " 8.00 | " 45.5 | 1.80 | 28 |
| 13 | " 8.00 | " 45.5 | 4.55 | 24 |
| 14 | " 8.00 | " 45.5 | 9.10 | 16 |
| 15 | $K_2CO_3$, 8.80 | " 45.5 | 0.23 | 10 |
| 16 | " 8.80 | " 45.5 | 0.46 | 20 |
| 17 | " 8.80 | " 45.5 | 1.72 | 8 |
| 18 | " 8.80 | " 45.5 | 2.30 | 4 |
| 19 | " 8.80 | " 45.5 | 3.44 | 8 |
| 20 | " 8.80 | " 45.5 | 5.16 | 16 |
| 21 | " 8.80 | " 45.5 | 9.17 | 15 |
| 22 | " 8.80 | " 45.5 | 17.20 | 13 |
| 23 | " 8.80 | " 45.5 | 22.90 | 10 |

The results illustrate how the rate of formation of formate in an unexpected way depends on the amount of water in the reaction mixture, and that the reaction is surprisingly solvent selective and sensitive to the specific alkali metal ion present. An evaluation and graphic representation of tests 5 and 15-23 are given on Figure 1. These results define how the molar ratio $H_2O/K_2CO_3$ (abscissa) must be in order to obtain high formation rates of potassium formate (ordinate). On the other hand, rubidium and cesium formate are formed at high rates in the whole range where the molar ratio between water and carbonate is less than 20, but above 0.2.

Example 6

This example illustrates how the individual steps may be combined to a cyclic process in which the reactions 1, 2 and 3 take place in separate vessels. With reference to Figure 2 the palladium catalyzed reaction 1 takes place in zone I, reaction 3 takes place in zone II, while the thermal decomposition of bicarbonate and removal of the by-product, $CO_2$, take place in zone III. Essentially pure hydrogen gas is recovered as product in flow 1. Carbon monoxide is injected into zone II via line 8 and is reacted therein with carbonate and water in the presence of methanol as solvent at a temperature in the range 100-150°C and a partial pressure of CO of about 10 bar. A mixture of formate, bicarbonate and carbonate leaves zone II via line 3 and is conveyed into zone III in which bicarbonate is transformed to $CO_2$ (which is recovered as by-product via line 9) water and carbonate at a temperature in the range 150180°C. Some of flow 4 which consists of methanol, water, formate and carbonate in addition to minor amounts of bicarbonate, is conveyed back to zone II via line 5 while some, via line 6, is mixed with water via line 7 and conveyed into zone I. Herein hydrogen is formed in accordance with reaction 1 above in the presence of the palladium catalyst (Pd(5%)/C) at a temperature in the range 100-150°C, and practically pure hydrogen is recovered as product via line 1. Unreacted formate, bicarbonate and carbonate are conveyed back to zone II via line 2. The concentration of salts is chosen so that they in all steps of the process are soluble in the liquid reaction medium which primarily consists of methanol and water. The concentration of water in zone II is adjusted so that the molar ratio between water and carbonate is within the limits mentioned above. Possible inert gases which are injected into zone II together with CO, are removed from the process via line 10.

Example 7

This example illustrates how the individual steps may be combined to a process in which the reactions 1 and 3 take place in the same vessel, while reaction 2 is carried out in a separate zone.

With reference to Figure 3, CO is fed into the process via line 1 and reacts primarily with carbonate and water in reactor I with the formation of formate at a temperature in the range 100-150°C in the presence of methanol as solvent. The molar ratio between water and carbonate is here as mentioned above. The formate thereby formed is then further reacted in the same reactor with water and Pd catalyst (Pd(5%)/C) to yield hydrogen and bicarbonate. Unreacted CO is recovered together with hydrogen as a product through line 2. The liquid reaction mixture consisting of formate, carbonate and bicarbonate dissolved in methanol/water, and undissolved Pd-catalyst (about 10 g per $dm^3$) are removed from the reactor I via line 3 and centrifuged. The Pd catalyst is recycled to reactor I via line 4, while the salt solution is conveyed into reactor II via line 5. In reactor II the bicarbonate is decomposed to carbonate, water and $CO_2$ at a temperature in the range 150-200°C. $CO_2$ is recovered as by-product via line 7. A mixture consisting primarily of carbonate and formate in methanol/water is then recycled to zone I via line 6. Water is injected into the process via line 8 at a rate which on molar basis is the same as the rate of formation of hydrogen.

Example 8

This example illustrates how the process may be performed when the reactions 2 and 3 take place in the same reactor, while reaction 1 takes place in a separate zone. With reference to Figure 4 CO is fed to the process via line 5 in reactor II, in which CO reacts with carbonate and water with primary formation of formate and bicarbonate at a temperature in the range 150-200°C in the presence of methanol as solvent. Then the bicarbonate reacts further in the same reactor to form carbonate, water and $CO_2$ which is separated from the process via line 4 together with minor amounts of unreacted CO. The formate solution formed in reactor II is mixed with water and conveyed via line 3 into reactor I in which it is contacted with a stationary phase of Pd catalyst, at a temperature in the range 100-150°C, whereby hydrogen gas is formed as a product and taken out of the process via line 1. The amount of methanol present in flow 1 is of course recycled to the process via line 2. The partial pressure of CO in reactor II is chosen so that a suitable reaction rate is obtained for the formation of formate in comparison with the amount of CO accompanying the byproduct, $CO_2$, in flow 4. Water is added to the process

via line 6 at such a rate that it on molar basis is the same as the formation of hydrogen.

Example 9

Catalytic water-gas shift reaction with in situ formation of formate

This example illustrates that all the reactions, 1-3, may take place simultaneously in the same reactor.

43.6 g of methanol, 8.75 g of $K_2CO_3$, 1.75 g of $H_2O$ and 0.3 g of Pd(5%)/activated carbon catalyst were charged to a 100 ml steel (316 SS) autoclave equipped with magnetic stirrer, electric heating means, temperature control means and mariometer (0.2-40 bar) at room temperature under a CO pressure of 10 bar. The gas volume in the apparatus was about 0.1 dm³. The reaction was starter by heating the liquid reaction mixture in closed apparatus to 150°C during 1 hour. The reaction temperature was thereafter kept constant at 149 ± 2°C. During the first 30 minutes of the heating period the pressure in the reactor dropped to 8.5 bar, and at the same time it was found that formate had been formed. The pressure then rose to 11.6 bar at a total reaction time of 92 min. A gas chromatographic analysis of the gas mixture above the liquid reaction mixture at this time showed that the composition thereof after cooling to room temperature was: 70% $H_2$, 26% CO and 4% $CO_2$.

In addition to potassium carbonate and potassium formate considerable amounts of potassium bicarbonate were also found in the cooled reaction mixture. It is well known that potassium bicarbonate may be converted to potassium carbonate by heating and removal of $CO_2$ according to known methods.

If desired, pure hydrogen may be recovered from the $H_2/CO/CO_2$ gas mixture by known processes, or the mixture may be used directly in syntheses.

Example 10

Example 9 was repeated with the only difference that the reaction temperature was 180 ± 2°C. The composition of the gas was in this case found to be: 60% $H_2$, 12% CO and 38% $CO_2$.

Example 11

Continuous production of hydrogen and carbon dioxide in one reactor.

With reference to Figure 5 a stoichiometric mixture of CO and water is fed continuously to a three phase bubble column reactor (liquid volume about 6 liters) via line 1 and is reacted therein via potassium formate and potassium bicarbonate as intermediates to hydrogen and $CO_2$ in the presence of a heterogeneous palladium catalyst, Pd(5%)/C (5 g/l), potassium carbonate, methanol and water at a temperature of 180°C. Unreacted CO is recycled via line 3 until the desired degree of conversion is obtained. The product is recovered continuously via line 2. At the start of the experiment the reaction mixture contains: 40 g $K_2CO_3$ per liter of methanol and an amount of water so that the molar ratio $H_2O/K_2CO_3$ is approximately 1. The reactor pressure is about 40 bar. In continuous operation there is recovered a gas mixture consisting of $H_2/CO_2$ in a molar ratio of 1/1 containing minor amounts of unreacted CO.

## Claims

1. Process for converting carbon monoxide and water to hydrogen and carbon dioxide, **characterized** in that potassium, rubidium or cesium bicarbonate is subjected to in situ or external thermal decomposition to liberate carbon dioxide, the potassium, rubidium or cesium carbonate respectively formed is reacted with carbon monoxide and water in a liquid reaction medium which comprises at least 50% by weight of methanol, at a temperature in the range 50-240°C and a carbon monoxide pressure in the range 1-50 bar, whereby potassium, rubidium or cesium formate respectively is formed in a liquid, aqueous methanol solution, and said solution is contacted in a reaction zone with a heterogeneous palladium catalyst which remains in said reaction zone, at a temperature in the range 50-240°C, whereby hydrogen is formed as product, and the simultaneously formed bicarbonate is recycled for continued use in the process.

2. Process according to claim 1, **characterized** in that the carbonate used is potassium carbonate, and the molar ratio of water:potassium carbonate is kept in the range 0.2-1.5 or 4 to 20.

3. Process according to claim 2, **characterized** in that the molar ratio of water:potassium carbonate is kept at about 1.

4. Process according to claim 1, **characterized** in that the carbonate used is rubidium or cesium carbonate,

and the molar ratio of water:carbonate is kept in the range 0.2-20.

## Patentansprüche

1. Verfahren zur Umwandlung von Kohlenstoffmonoxid und Wasser in Wasserstoff und Kohlenstoffdioxid, dadurch gekennzeichnet, daß Kalium-, Rubidium- oder Cäsiumbicarbonat einer in situ oder extern erfolgenden thermischen Zersetzung unter Freisetzung von Kohlenstoffdioxid unterworfen wird, das gebildete Kalium-, Rubidium- oder Cäsiumcarbonat in einem flüssigen Reaktionsmedium, das mindestens 50 Gew.-% Methanol enthält, bei einer Temperatur im Bereich von 50 bis 240°C und einem Kohlenstoffmonoxiddruck im Bereich von 1 bis 50 bar mit Kohlenstoffmonoxid und Wasser umgesetzt wird, wobei Kalium-, Rubidium- oder Cäsiumformiat in einer flüssigen, wäßrigen Methanollösung gebildet wird, und diese Lösung bei einer Temperatur im Bereich von 50 bis 240°C in einer Reaktionszone mit einem heterogenen Palladiumkatalysator, der in dieser Reaktionszone verbleibt, kontaktiert wird, wobei als Produkt Wasserstoff gebildet wird, und das gleichzeitig gebildete Bicarbonat zur kontinuierlichen Verwendung in dem Verfahren recyclisiert wird.

2. Verfahren gemäß Anspruch 1, dadurch gekennzeichnet, daß das verwendete Carbonat Kaliumcarbonat ist und das Molverhältnis Wasser:Kaliumcarbonat im Bereich von 0,2 bis 1,5 oder 4 bis 20 gehalten wird.

3. Verfahren gemäß Anspruch 2, dadurch gekennzeichnet, daß das Molverhältnis Wasser:Kaliumcarbonat bei etwa 1 gehalten wird.

4. Verfahren gemäß Anspruch 1, dadurch gekennzeichnet, daß das verwendete Carbonat Rubidium- oder Cäsiumcarbonat ist und daß das Molverhältnis Wasser:Carbonat im Bereich von 0,2-20 gehalten wird.

## Revendications

1. Procédé de conversion de monoxyde de carbone et d'eau en hydogène et en dioxyde de carbone, caractérisé en ce que l'on soumet du bicarbonate de potassium, de rubidium ou de césium à une décomposition thermique, in situ ou externe, afin de libérer du dioxyde de carbone, on fait réagir respectivement le carbonate de potassium, de rubidium ou de césium ainsi formé avec du monoxyde de carbone et de l'eau dans un milieu réactionnel liquide qui comprend au moins 50% en poids de méthanol, à une température qui varie de 50 à 240°C et à une pression de monoxyde de carbone qui fluctue de 1 à 50 bars, en sorte que se forme respectivement du formiate de potassium, de rubidium ou de césium dans une solution aqueuse de méthanol, liquide, et on met cette solution en contact, dans une zone de réaction, avec un catalyseur au palladium hétérogène, qui demeure dans la zone de réaction, à une température qui varie de 50 à 240°C, si bien que de l'hydrogène se forme à titre de produit et que le bicarbonate simultanément formé est recyclé en vue de sa mise en oeuvre continue dans le procédé.

2. Procédé suivant la revendication 1, caractérisé en ce que le carbonate utilisé est le carbonate de potassium et le rapport molaire eau:carbonate de potassium est maintenu dans la plage de 0,2-1,5 ou 4 à 20.

3. Procédé suivant la revendication 2, caractérisé en ce que le rapport molaire eau:carbonate de potassium est maintenu à environ 1.

4. Procédé suivant la revendication 1, caractérisé en ce que le carbonate utilisé est le carbonate de rubidium ou de césium et le rapport molaire eau:carbonate est maintenu dans la plage de 0,2 à 20.

Fig. 1

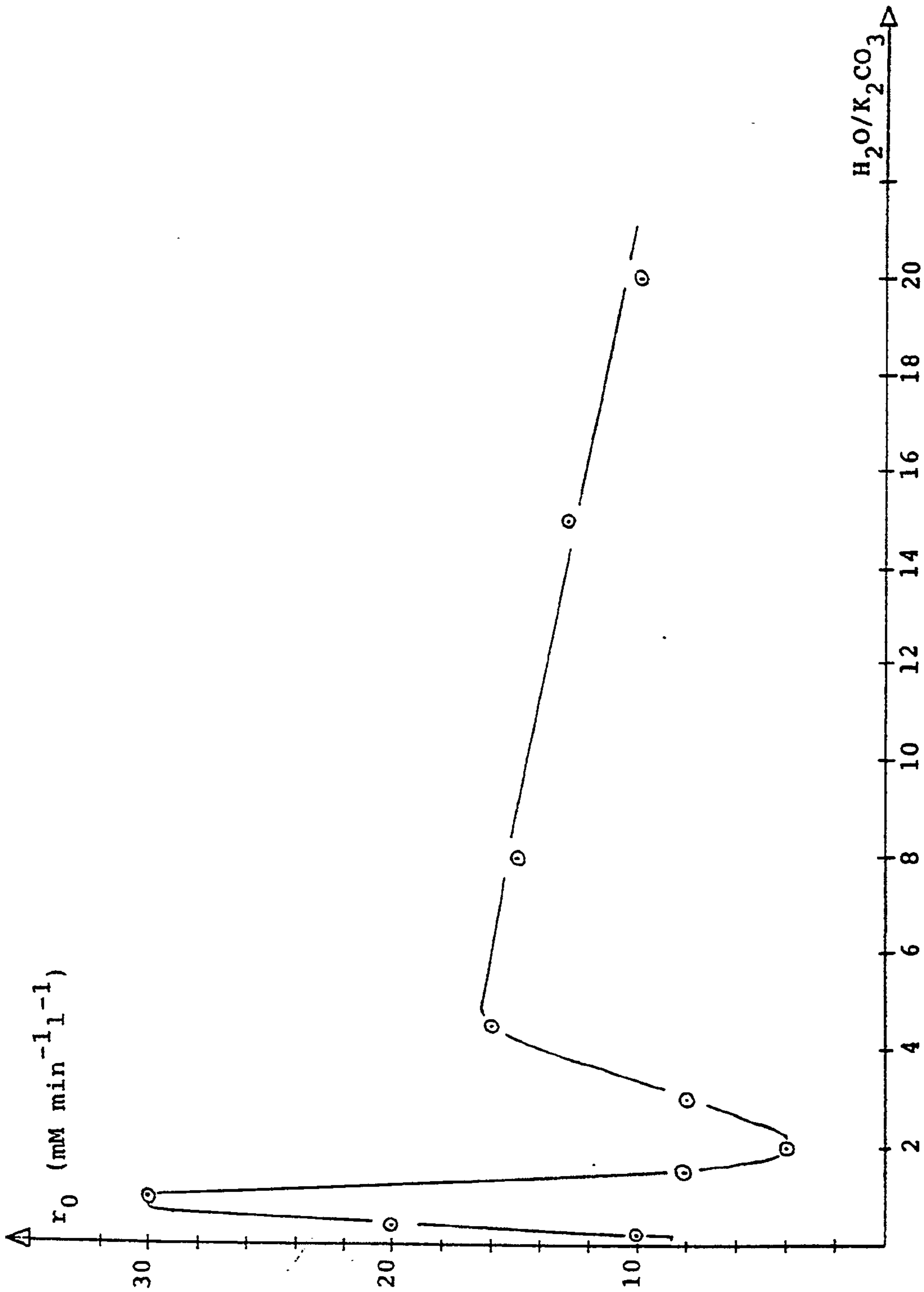

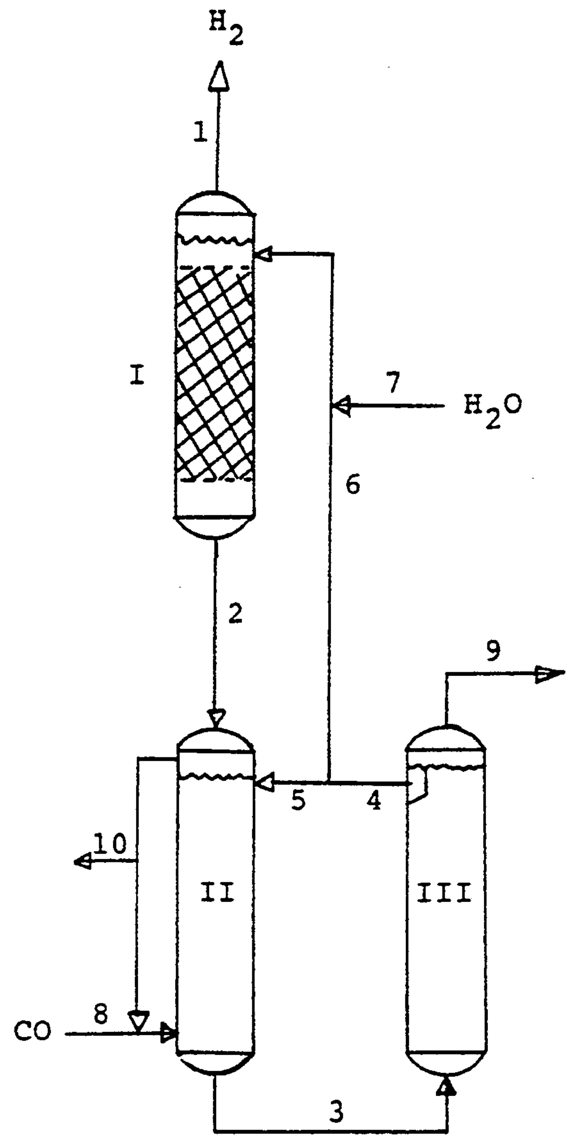


Fig. 2

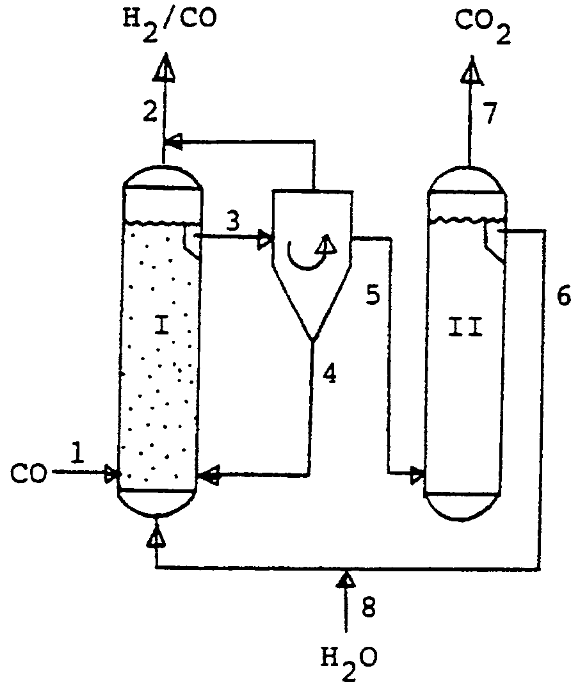


Fig. 3

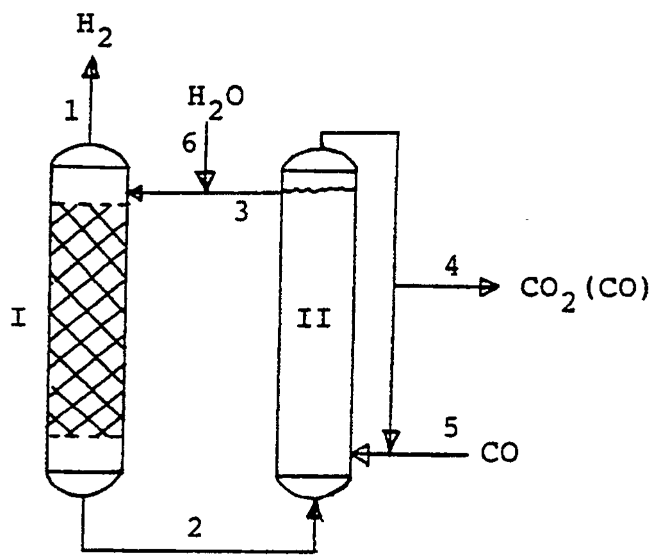


Fig. 4

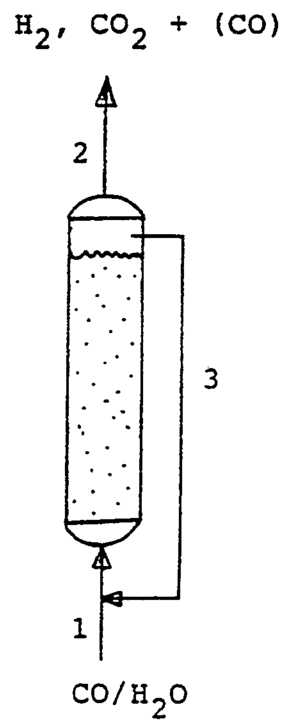


Fig. 5